(19) Europäisches Patentamt
European Patent Office
Office européen des brevets

(11) **EP 1 172 110 A2**

(12) **EUROPÄISCHE PATENTANMELDUNG**

(43) Veröffentlichungstag:
**16.01.2002 Patentblatt 2002/03**

(21) Anmeldenummer: **01116197.3**

(22) Anmeldetag: **04.07.2001**

(51) Int Cl.[7]: **A61K 33/00**, A61P 35/00, A61P 31/18, A61P 13/00, A61P 3/00, A61P 1/00 // (A61K33/00, 31:385)

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **07.07.2000 DE 10032601**
**26.02.2001 DE 10109303**

(71) Anmelder: **BASF AKTIENGESELLSCHAFT**
**67056 Ludwigshafen (DE)**

(72) Erfinder:
- **Krämer, Klaus, Dr.**
  **76829 Landau (DE)**
- **Klatt, Martin, Jochen, Dr.**
  **67098 Bad Dürkheim (DE)**

(54) **Verwendung von Liponsäure zur Verbesserung der Bioverfügbarkeit von Mineralsalzen**

(57) Die vorliegende Erfindung betrifft die Verwendung von α-Liponsäure oder α-Dihydroliponsäure zur Erhöhung der Bioverfügbarkeit von Mineralsalzen, die Verwendung von α-Liponsäure oder α-Dihydroliponsäure in Kombination mit Metallsalzen, insbesondere die Verwendung von Metall-α-Lipoaten, Metall-α-Dihydrolipoaten oder Metall-α-Liponsäure-Komplexen, insbesondere in Mineralstoffzubereitungen oder Arzneimitteln sowie die Metall-α-Lipoate, Metall-α-Dihydrolipoate oder Metall-α-Liponsäure-Komplexe selbst.

EP 1 172 110 A2

## Beschreibung

Die vorliegende Erfindung betrifft die Verwendung von α-Liponsäure oder α-Dihydroliponsäure zur Erhöhung der Bioverfügbarkeit von Mineralsalzen, die Verwendung von α-Liponsäure oder α-Dihydroliponsäure in Kombination mit Metallsalzen, insbesondere die Verwendung von Metall-α-Lipoaten, Metall-α-Dihydrolipoaten oder Metall-α-Liponsäure-Komplexen, insbesondere in Mineralstoffzubereitungen oder Arzneimitteln sowie die Metall-α-Lipoate, Metall-α-Dihydrolipoate oder Metall-α-Liponsäure-Komplexe selbst.

Mineralstoffe sind die Bestandteile pflanzlicher und tierischer Gewebe, die bei der Verbrennung als Asche zurückbleiben. Nach dem Anteil der einzelnen Elemente teilt man die Mineralstoffe in Mengenelemente wie beispielsweise Ca, P, K, Cl, Na, Mg oder in Spurenelemente, wie beispielsweise Fe, Zn, Cu, Mn, V, Se, Mn, J, Sn, Ni, Mo, Cr, Co ein.

Die metallischen Mineralstoffe werden als Kationen, in der Regel als Mineralsalze mit anorganischen Anionen vom menschlichen oder tierischen Organismus aufgenommen. Die essentiellen metallischen Mineralstoffe mit bekannter biologischer Funktion im Organismus haben im Organismus sehr vielfältige Funktionen, beispielsweise als Elektrolyte, Bestandteile von Enzymen oder als Bausteine bestimmter Körpersubstanzen.

Mineralsalze zur Supplementation bzw. Therapie von Mineralstoffmangelzuständen in menschlichen oder tierischen Organismen sollten eine möglichst hohe Bioverfügbarkeit aufweisen.

Es ist bekannt, daß möglicherweise die Bioverfügbarkeit von Zinksalzen durch Komplexierung mit Proteinat gegenüber $ZnSO_4$ erhöht werden kann (B.A. Reiling et al, J. of Animal Science 1992, 70, Supplement 1, 84th Annual Meeting, Abstract 649).

Ferner ist bekannt, daß die Komplex- oder Salzbildung von Zinksalzen mit Methionin zu einer erhöhten Bioverfügbarkeit von $Zn^{2+}$ bei Hühnern führt (Wedekind, J. Anim. Sci. 1992, 70, S. 178).

Dahingegen zeigte sich im Schweinemodell, daß die Bioverfügbarkeit der organischen Salze von Zink mit Methionin oder Lysin schlechter ist, als die des anorganischen $ZnSO_4 \cdot H_2O$ (Wedekind et al., J. Anim. Sci 1994, 72 S. 2681 bis 2689).

Weitere Untersuchungen mit $ZnCl_2$, ZnMet und Zinkpropionat ergaben eine etwa vergleichbare Bioverfügbarkeit von $Zn^{2+}$ aus organischen oder anorganischen Quellen (Beutler et al., Biological Trace Element research, 1998, 61, Seite 19), wenn auch Rojas et al. eine leicht erhöhte Bioverfügbarkeit der organischen Zinksalze ZnMet oder ZnLys gegenüber $ZnSO_4$ in Schafen feststellen konnte (J. Anim. Sci. 1995, 73, S. 1202 bis 1207).

Für Citronensäure wird ein positiver Effekt auf die Zn-Bioverfügbarkeit beschrieben. So wurde bei wachsenden Ratten bei suboptimaler alimentärer Zn-Versorgung durch die Zulage von 1 % Citronensäure zu phytinsäurereichen Diäten auf der Basis von Maiskeimen, eine moderate Verbesserung des Zn-Status (Pläsma-Zn-Konzentration, Aktivität der alkalischen Phosphatase, Metallothronein-Konzentration im Jejunum) erzielt (Pallauf et al, Z. Ernährungswiss 1990, 29, Seite 27 bis 38).

Vergleichbar positive Effekte der Citronensäure auf die Zn-Verwertung wurden auch bei Schweinen gefunden (Pallauf et al., J. Anim. Physiol. a. Anim. Nutr. 1994, 71, Seite 189 bis 199).

Ascorbinsäure erhöht die Bioyerfügbarkeit von anorganischem Eisen (Hallberg et al., Hum. Nutr. Appl. Nutr. 1986, 40A, 97 bis 113). Im Gegensatz dazu wird durch Ascorbinsäure die Bioverfügbarkeit des Nahrungszinks nicht oder nur sehr unwesentlich verändert (Sandström und Lederblad, Int. J. Vitamin Nutr. Res 1987, 57, 87 bis 90, Solmons et al. Am. J. Clin. Nutr. 1979, 32, 2495 bis 2499).

Die Ergebnisse bisheriger Studien zum Einfluß von Oxalsäure (Kelsay et al, Am. J. Clin. Nutr. 1983, 31, 1198 bis 1203; Welch et al. J. Nutr. 1977, 107, 929 bis 933) und Picolinsäure (Schwarz et al., Res. Exp. Med. 1983, 182, 39 bis 48; Seal et al. J. Nutr. 1985, 115, 986 bis 993) auf die Zn-Bioverfügbarkeit sind uneinheitlich und lassen keine eindeutigen Schlußfolgerungen zu.

Die im Stand der Technik verwendeten Metallkomplexe bzw. salze oder Mineralsalze und organische Chelatoren führen also nur bedingt zu einer erhöhten Bioverfügbarkeit, so daß ein hoher Bedarf an neuen Verbindungen bzw. an neuen organischen Chelatoren oder Salzen besteht, die zu einer erhöhten Bioverfügbarkeit der Mineralkationen verglichen mit den anorganischen Mineralsalzen führen.

Der Erfindung lag daher die Aufgabe zugrunde neue organische Chelatoren bzw. Komplex- oder Salzbildner sowie neue organische Mineralverbindungen zur Verfügung zu stellen, die gegenüber den anorganischen Mineralsalzen eine erhöhte Bioverfügbarkeit, weniger Nachteile als der Stand der Technik und weitere Vorteile aufweisen.

Demgemäß wurde gefunden, daß α-Liponsäure oder α-Dihydroliponsäure zur Erhöhung der Bioverfügbarkeit von Mineralsalzen verwendet werden kann.

Unter Bioverfügbarkeit einer Verbindung wird in der vorliegenden Erfindung

der Anteil einer Verbindung verstanden, der nach oraler Gabe im Darm absorbiert wird und durch die Leber unverändert im Blutkreislauf erscheint oder

die intestinale Verdauung und Absorption einer Verbindung, also die scheinbaren Absorption bzw. unter Berücksichtigung der sogenannten endogenen Verluste, die wahre Absorption, wobei bei der Bestimmung der wahren Absorption

gegenüber der scheinbaren Absorption die Menge einer Verbindung berücksichtigt wird, die bereits zu einem früheren Zeitpunkt absorbiert wurde und durch Sekretionsmechanismen und Darmabschilferungen (endogene Verluste) ebenfalls mit den Faeces ausgeschieden wird bzw. nach Korrektur um die renale Ausscheidung eine Bilanz ermittelt wird oder

eine Sequenz von metabolischen Ereignissen, die neben Verdauung, Löslichkeit, Absorption und Verteilung die Organaufnahme und -abgabe sowie enzymatische Transformation, Sekretions- und Exkretionsmechanismen beinhaltet und damit metabolische Funktionen, in Form von Cofaktoren für Enzyme und Hormone bzw. Stabilisierung von Membranlipid und Strukturproteinen, übernehmen kann. beispielsweise dadurch gekennzeichnet, daß enzymatische Aktivitäten erhöht werden oder

die Akkumulation einer Verbindung in Organen wie Leber und Knochen.

Erfindungsgemäß wird unter einer erhöhten Bioverfügbarkeit eines Mineralsalzes eine Verbesserung in mindestens einem der vorstehend beschriebenen Parameter der Bioverfügbarkeit, verglichen mit einem anorganischen Mineralsalzes, wie beispielsweise einem Sulfat oder Halogenid, verstanden.

Unter α-Liponsäure werden erfindungsgemäß racemische α-Liponsäure oder α-Lipoate oder enantiomerenreine (R)- oder (S)-α-Liponsäure oder (R)- oder (S)-α-Lipoate, unter α-Dihydroliponsäure racemische α-Dihydroliponsäure oder α-Dihydrolipoate oder enantiomerenreine (R)- oder (S)-α-Dihydroliponsäure oder (R)-oder (S)-α-Dihydrolipoate verstanden.

Unter "racemisch" wird nicht nur eine 1:1 Mischung beider Enantiomere sondern auch angereicherte Enantiomere die in unterschiedlichen Verhältnissen vorliegen können, wie beispielsweise in einem Verhältnis von 99:1, verstanden.

In einer bevorzugten Ausführungsform wird die Bioverfügbarkeit von Mineralsalzen dadurch erhöht, daß man mindestens ein Mineralsalz in Kombination mit α-Liponsäure oder α-Dihydroliponsäure verwendet.

Mineralsalze sind Salze physiologisch verträglicher, ein bis dreiwertiger Metalle.

Bevorzugte Mineralsalze, deren Bioverfügbarkeit in Kombination mit α-Liponsäure oder α-Dihydroliponsäure erhöht wird, sind Mineralsalze der Formel I,

$$(M)_n(B)_m \qquad \text{I}$$

wobei

M ein 1 bis 3-wertiges, physiologisch verträgliches Metallkation,

B ein 1 bis 3-wertiges, physiologisch verträgliches Anion,

n 1, 2 oder 3 und

m 1, 2 oder 3 bedeuten,

wobei die Indizes n und m dem Wertigkeits- und Ladungsausgleich des Mineralsalzes der Formel I entsprechen.

Bevorzugte physiologisch verträgliche, ein bis dreiwertige Metallkation M sind die essentiellen Metallkationen, wie beispielsweise $Na^+$, $K^+$, $Mg^{2+}$, $Ca^{2+}$, $Fe^{2+}$, $Fe^{3+}$, $Sn^{2+}$, $Zn^{2+}$, $Cu^{2+}$, $Mn^{2+}$, $Mn^{3+}$, $Cr^{3+}$, $Mo^{3+}$, $Co^{2+}$ oder $Ni^{2+}$.

Bevorzugte physiologisch verträgliche, ein bis dreiwertige Anionen B sind beispielsweise anorganische Anionen, insbesondere Halogenide wie $F^-$ oder $Cl^-$, Chalkogenide, wie beispielsweise $O^{2-}$ oder die Anionen, $NO_3^-$, $SO_4^{2-}$, $CO_3^{2-}$, $PO_4^{3-}$, $HCO_3^-$, $HPO_4^{2-}$, $H_2PO_4^-$ oder die organischen Anionen Ascorbat, Oxalat, Citrat, Gluconat, Picolinat, Aspartat, Histidinat, Saccharat, Orotat, Lactobionat, Lactat, Fumarat, Formiat, Acetat, Glucobionat, Glucocephat oder auch beispielsweise die nachstehend beschriebenen Anionen α-Lipoat oder α-Dihydrolipoat.

Durch die kombinierte Verwendung mindestens eines Mineralsalzes mit α-Liponsäure oder α-Dihydroliponsäure wird die Bioverfügbarkeit des Mineralsalzes erhöht.

Unter Kombination wird die gleichzeitige oder zeitlich und/oder örtlich versetzte Gabe mindestens eines Mineralsalzes und α-Liponsäure oder α-Dihydroliponsäure verstanden. Beispielsweise können mindestens ein Mineralsalz und α-Liponsäure oder α-Dihydroliponsäure in einer Formulierung, wie beispielsweise einer Mineralstoffzubereitung oder einer Arzneimittelzubereitung gemeinsam verabreicht werden.

In einer besonders bevorzugten Ausführungsform verwendet man in der vorstehend beschriebenen Kombination mit Mineralsalzen, vorzugsweise Mineralsalzen der Formel I α-Liponsäure, insbesondere (R)-α-Liponsäure.

Die Erfindung betrifft ferner eine Zubereitung, insbesondere ein Mineralstoffzubereitung oder eine Arzneimittelzubereitung, enthaltend mindestens ein Mineralsalz und (R)-α-Liponsäure oder (S)-α-Liponsäure, vorzugsweise

(R)-α-Liponsäure. Vorzugsweise beträgt der Anteil des einen Enantiomer bei Anwesenheit des anderen Enantiomer mindestens 70 Mol-% gegenüber dem anderen Enantiomer.

Eine Mineralstoffzubereitung kann beispielsweise weitere Mineralsalze, Formulierungshilfsstoffe sowie gegebenenfalls auch Vitamine oder Vitaminmischungen enthalten.

Eine Arzneimittelzubereitung kann beispielsweise weitere Wirkstoffe sowie Hilfsstoffe, wie beispielsweise Füllstoffe, Konservierungsmittel, Tablettensprengmittel, Fließreguliermittel, Weichmacher, Netzmittel, Dispergiermittel, Emulgatoren, Lösungsmittel, Retardierungsmittel oder Antioxidantien enthalten (vgl. H. Sucker et al.: Pharmazeutische Technologie, Thieme-Verlag, Stuttgart, 1991).

Die Mengen in denen die α-Liponsäure oder α-Dihydroliponsäure im Verhältnis zu einem Mineralsalz eingesetzt wird, ist nicht kritisch und richtet sich nach der physiologisch verträglichen Menge. Typischerweise wird die α-Liponsäure oder α-Dihydroliponsäure im molaren Verhältnis von 0,1:1 bis 1000:1 zu einem Mineralsalz eingesetzt.

In einer bevorzugten Ausführungsform verwendet man als Kombination, also als Mineralsalz mit erhöhter Bioverfügbarkeit, direkt Metall-α-Lipoate, Metall-α-Dihydrolipoate oder Metall-α-Liponsäure-Komplexe.

Diese Verbindungen weisen eine erhöhte Bioverfügbarkeit auf.

Diese Verbindungen haben den weiteren Vorteil, daß der metallische Mineralstoff und die therapeutisch wertvolle Liponsäure in einer Formulierung vorliegen. Diese Verbindungen eignen sich deshalb bevorzugt als raumsparende Inhaltsstoffe in kosmetischen Formulierungen, in Arzneimittelformulierungen sowie in Formulierungen von Futter- und Nahrungsergänzungsmitteln, insbesondere in festen Darreichungsformen.

In einer bevorzugten Ausführungsform können die Metall-α-Lipoate, Metall-α-Dihydrolipoate oder Metall-α-Liponsäure-Komplexe der Formel II als Mineralstoffe mir einer erhöhten Bioverfügbarkeit verwendet werden,

$$(M)_w(Lp)_x(A)_y(H_2O)_z \qquad \text{II}$$

wobei

- M ein 1 bis 3-wertiges, physiologisch.verträgliches Metallkation oder ein Gemisch 1- bis 3-wertiger, physiologisch verträglicher Metallkationen,
- Lp racemische α-Liponsäure oder α-Dihydroliponsäure, (R)- oder (S)-α-Liponsäure oder (R)- oder (S)-α-Dihydroliponsäure, racemisches α-Lipoat oder Dihydro-α-lipoat oder (R)- oder (S)-α-Lipoat oder (R)- oder (S)-Dihydro-α-lipoat,
- A ein physiologisch verträgliches, ein oder zweiwertiges Anion,
- w 1 oder 2
- x 1, 2, 3 oder 4,
- y 0, 1, 2 oder 3 und
- z 0, 1, 2, 3, 4, 5 oder 6, bedeuten,

wobei die Indizes w, x und y dem Wertigkeits- und Ladungsausgleich entsprechen. Bevorzugte physiologisch verträgliche, ein bis dreiwertige Metallkation M sind, wie vorstehend beschrieben, die essentiellen Metallkationen, wie beispielsweise $Na^+$, $K^+$, $Mg^{2+}$, $Ca^{2+}$, $Fe^{2+}$, $Fe^{3+}$, $Sn^{2+}$, $Zn^{2+}$, $Cu^{2+}$, $Mn^{2+}$, $Mn^{3+}$, $Cr^{3+}$, $Mo^{3+}$, $Co^{2+}$ oder $Ni^{2+}$, insbesondere $Zn^{2+}$, $Mg^{2+}$, $Fe^{2+}$, $Fe^{3+}$, $Ca^{2+}$, $Mn^{3+}$ oder $Cr^{3+}$.

Die α-Liponsäure oder deren reduzierte Form, die α-Dihydroliponsäure gehen mit Metallen als Anionen (α-Lipoat: einwertig negativ; α-Dihydrolipoat: einwertig oder zweiwertig negativ) salzartige Ionenbindungen und/oder koordinative Bindungen über den Carboxylsauerstoff oder die geschlossene bzw. offene Disulfideinheit ein. Die α-Liponsäure oder die α-Dihydroliponsäure kann auch als neutrales Molekül an Metallkationen koordinativ gebunden sein. Sie können sowohl als Racemat als auch enantiomerenrein in der (R)- oder (S)-Form eingesetzt werden.

Bevorzugte physiologisch verträgliche, ein bis zweiwertige Anionen A sind beispielsweise anorganische Anionen, insbesondere Halogenide wie $F^-$ oder Cl-, Chalkogenide, wie beispielsweise $O^{2-}$ oder die Anionen, $NO_3^-$, $SO_4^{2-}$, $CO_3^{2-}$, $HCO_3^-$, $HPO_4^{2-}$, $H_2PO_4^-$ oder die organischen Anionen Ascorbat, Oxalat, Citrat, Gluconat, Picolinat, Aspartat, Histidinat, Saccharat, Orotat, Lactobionat, Lactat, Fumarat, Formiat, Acetat, Glucobionat oder Glucocephat

In den Verbindungen der Formel II können bis zu 6 Moleküle Wasser, vorzugsweise bis zu 4 Moleküle Wasser,

insbesondere bis zu zwei Moleküle Wasser koordinativ gebunden sein.

Bei bevorzugten Verbindungen der Formel II werden als Rest Lp α-Lipoate verwendet, insbesondere (R)-α-Lipoate und der Anteil an weiteren Anionen A ist 0 (y = 0). In dieser bevorzugten Ausführungsform entsprechen die Indizes w und x dem Wertigkeits-und Ladungsausgleich der Verbindungen der Formel II.

Es kann vorteilhaft sein die Verbindungen der Formel II in Kombination mit α-Liponsäure oder α-Dihydroliponsäure, beispielsweise zur weiteren Steigerung der Bioverfügbarkeit, zu verwenden.

Die Erfindung betrifft daher weiterhin eine Zubereitung enthaltend Verbindungen der Formel II, vorzugsweise Verbindungen der Formel II' und α-Liponsäure oder α-Dihydroliponsäure.

Siegel et al. beschreiben in strukturellen Arbeit binäre Komplexe von Mn, Cu, Zn, Cd und Pb mit racemischen und (R)-und (S)-Lipoaten (Archives of Biochemistry and Biophysics 1978, 187, Seite 208 bis 214; Angew. Chem. 1982, 94, 421-432).

P.R. Brown et al. beschreiben in weiteren strukturellen Arbeiten einen binären Komplex von Hg mit racemischer α-Liponsäure,
wobei die Liponsäure als neutrales Molekül koordinativ gebunden ist. Weiterhin werden binäre Komplexe von Hg und Ni mit racemischem, zweiwertig negativem α-Dihydrolipoat beschrieben. Die strukturellen Aufklärungen dienten zur Untersuchung der Verwendung von Liponsäure zur Behandlung von Schwermetallvergiftungen (J. Inorg. Nucl. Chem. 1970, 32, 2671 bis 2675).

Bonomi et al. beschreiben einen Komplex von Fe mit racemischer α-Dihydroliponsäure zur Untersuchung der Verwendung von Dihydroliponsäure zum Entfernen von Ferritingebundenem Eisen (Biochemica et Biophysica Acta 1989, 994, 180 bis 186).

In einer weiteren strukturellen Arbeit werden von Strasdeit et al. Komplexe von Zn und Cd mit einwertig negativem, racemischen α-Lipoaten beschrieben, wobei zwei Moleküle Wasser an das Zentralatom koordiniert sind (Z. Naturforsch 1997, 52b, 17 bis 24).

Die Erfindung betrifft daher die neuen Metall-α-Lipoate, Metall-α-Dihydrolipoate oder Metall-α-Liponsäure-Komplexe der Formel II'

$$(M)_w(Lp)_x(A)_y(H_2O)_z \qquad II'$$

wobei

M ein 1 bis 3-wertiges, physiologisch verträgliches Metallkation oder ein Gemisch 1- bis 3-wertiger, physiologisch verträglicher Metallkationen,

Lp racemische α-Liponsäure oder α-Dihydroliponsäure, (R)- oder (S)-α-Liponsäure oder (R)- oder (S)-α-Dihydroliponsäure, racemisches α-Lipoat oder Dihydro-α-lipoat oder (R)- oder (S)-α-Lipoat oder (R)- oder (S)-Dihydro-α-lipoat,

A ein physiologisch verträgliches, ein oder zweiwertiges Anion,

w 1 oder 2,

x 1, 2, 3 oder 4,

y 0, 1, 2 oder 3 und

z 0, 1, 2, 3, 4, oder 6, bedeuten,

wobei die Indizes w, x und y dem Wertigkeits- und Ladungsausgleich entsprechen und
folgende Verbindungen ausgenommen sind:
$Mn(Lip^-)ClO_4$, $Cu(Lip^-)ClO_4$, $Zn(Lip^-)ClO_4$, $Cd(Lip^-)ClO_4$, $Pb(Lip^-)ClO_4$, $Hg(Lip_{rac})\ (OH)_2$, $Hg(DHL_{rac}{}^{2-})$, $Ni(DHL_{rac}{}^{2-})$, $Fe_2(DHL_{rac}{}^{2-})_3$, $Zn(Lip_{rac}{}^-)_2(H_2O)_2$, $Cd(Lip_{rac}{}^-)_2(H_2O)_2$
wobei

Lip - einwertig negatives, racemisches oder (R)- oder (S)-α-Lipoat,
$Lip_{rac}$ - einwertig negatives, racemisches α-Lipoat,
$Lip_{rac-}$ racemische α-Liponsäure und

$DHL_{rac}^{2}$ - zweiwertig negatives, racemisches α-Dihydrolipoat bedeuten.

Die Verbindungen der Formel II' entsprechen, auch in den bevorzugten Ausführungsformen, bis auf die ausgenommenen Verbindungen den vorstehend beschriebenen Verbindungen der Formel II.

Die Erfindung betrifft ferner eine Zubereitung, insbesondere eine Mineralstoffzubereitung oder eine Arzneimittelzubereitung, enthaltend Metall-α-Lipoate, Metall-α-Dihydrolipoate oder Metall-α-Liponsäure-Komplexe der Formel II'.

Eine Mineralstoffzubereitung kann beispielsweise weitere Mineralsalze, Formulierungshilfsstoffe, wie beispielsweise Füllstoffe, Farbstoffe sowie gegebenenfalls auch Vitamine oder Vitaminmischungen enthalten. Die Dosierung der einzelnen Komponenten richten sich nach dem gewünschten Gehalt, der sich beispielsweise an dem empfohlenen Tagesbedarf orientieren kann und je nachdem ob die Mineralstoffzubereitung beispielsweise als Nahrungsergänzung, Nahrungsersatz oder Futtermittelergänzung verwendet werden soll.

Eine Arzneimittelzubereitung kann beispielsweise weitere Wirkstoffe sowie Hilfsstoffe, wie beispielsweise Füllstoffe, Konservierungsmittel, Tablettensprengmittel, Fließreguliermittel, Weichmacher, Netzmittel, Dispergiermittel, Emulgatoren, Lösungsmittel, Retardierungsmittel oder Antioxidantien, sowie auch an sich bekannte Hilfsstoffe für eine parenterale oder enterale Applikation enthalten (vgl. H. Sucker et al.: Pharmazeutische Technologie, Thieme-Verlag, Stuttgart, 1991).

Die Erfindung betrifft ferner die Verwendung der Metall-α-Lipoate, Metall-α-Dihydrolipoate oder Metall-α-Liponsäure-Komplexe der Formel II als Futter- oder Nahrungsergänzungsmittel.

Die Verbindungen der Formel II stellen darüberhinaus in Einem eine antioxidative Wirkung und eine Versorgung an Mineralstoffen bereit. Sie können somit auch in kosmetischen Formulierungen Anwendung finden.

Daher betrifft die Erfindung ferner die Verwendung der Verbindungen der Formel II in kosmetischen Formulierungen.

Ferner betrifft die Erfindung die Metall-a-Lipoate, Metall-α-Dihydrolipoate oder Metall-α-Liponsäure-Komplexe der Formel II zur Verwendung als Arzneimittel.

Die Metall-α-Lipoate, Metall-α-Dihydrolipoate oder Metall-α-Liponsäure-Komplexe der Formel II können zur Herstellung eines Arzneimittels zur Behandlung von Krankheiten verwendet werden, bei denen Liponsäure einen therapeutischen oder prophylaktischen Effekt hat und ein Mineralsalzmangel vorliegt.

Beispielsweise wirkt α-Liponsäure, insbesondere (R)-α-Lipon-säure als Insulinsensitizer bei der Prävention und Therapie von Diabetes mellitus. Darüberhinaus wird Liponsäure zur Therapie der diabetischen Polyneuropathi eingesetzt. Bei Diabetikern tritt häufig ein Mineralsalzmangel, insbesondere ein Zinkmangel auf.

Die Metall-α-Lipoate, Metall-α-Dihydrolipoate oder Metall-α-Liponsäure-Komplexe der Formel II können somit zur Behandlung von Diabetis, darüberhinaus zur Behandlung von Tumorerkrankungen, HIV-Infektionen, AIDS, Niereninsuffizienz, Mangelernährung, Proteinenergiemalnutrition sowie von Mineralstoffmangelzuständen.

Mineralstoffmangelzustände können beispielsweise als Folge von Fehlernährung, einseitiger Ernährung, Arzneimitteleinahme, wie beispielsweise die Einnahme von Diurethika, Diarrhöe, Alkoholkonsum, parenterale oder enterale Ernährung, Trauma(OP), Blutverlust oder Antagonismen von anderen Nahrungsinhaltsstoffen, wie beispielsweise Phytat, Ballaststoffe, Oxalat- oder Phosphatüberschuß.

Die Metall-α-Lipoate, Metall-α-Dihydrolipoate oder Metall-α-Liponsäure-Komplexe der Formel II sind in an sich bekannter Weise, beispielsweise wie in Siegel et al., Archives of Biochemistry and Biophysics 1978, 187, Seite 208 bis 214; Angew. Chem. 1982, 94, 421-432; P.R. Brown et al., J. Inorg. Nucl. Chem. 1970, 32, 2671 bis 2675; Bonomi et al., Biochemica et Biophysica Acta 1989, 994, 180 bis 186 und Strasdeit et al., Z. Naturforsch 1997, 52b, 17 bis 24 herstellbar.

Vorzugsweise erfolgt die Herstellung durch Umsetzen von Alkalimetallsalzen der α-Liponsäure oder der α-Dihydroliponsäure in Lösung, vorzugsweise in wäßrig methanolischer Lösung, mit Mineralsalzen und anschließender Kristallisation.

Die Verwendung von α-Liponsäure oder α-Dihydroliponsäure zur Erhöhung der Bioverfügbarkeit von Mineralsalzen sowie die erfindungsgemäßen Verbindungen weisen im Vergleich mit herkömmlichen anorganischen Mineralquellen noch weitere Vorteile auf, wie

die Steigerung des Lebendmassezuwachs und der Futter-/Nahrungsaufnahme von Probanden, insbesondere von Tieren,

die Erhöhung der Mineralsalzverfügbarkeit und des Mineralsalzstatus, insbesondere die Erhöhung der Mineralsalzaufnahme, die Erhöhung der absoluten scheinbaren Absorption und der scheinbaren Absorption von Mineralsalzen, die Erhöhung der Mineralsalzeinlagerung in Femurgewebe und der Mineralsalz-Konzentration im Plasma, die Erhöhung der Aktivität der alkalischen Phosphatase und die Steigerung der hepatischen Metallothiomein-Konzentration.

Die nachstehenden Beispiele erläutern die Erfindung:

I. Synthesebeispiele

Beispiel 1

Synthese von Zn((R)-α-Lipoat)$_2$($H_2O$)$_2$

2,06 g (10 mmol) (R)-α-Liponsäure wurden in 150 ml Methanol gelöst und bei Raumtemperatur mit einer Lösung aus 0,4 g (10 mmol) NaOH in 50 ml Wasser unter Rühren versetzt.
1,49 g (5 mmol) Zinknitrat wurden in 150 ml Methanol schnell zur Lösung des Natriumsalzes dosiert und die Lösung weitere zwei Stunden gerührt.
Die klare, blaßgelbe Lösung wurde in eine Petrischale überführt. Nach Abdampfen des Lösungsmittels erhielt man einen gelben Niederschlag, der sorgfältig mit Wasser und Toluol gewaschen und über Nacht im Stickstoffgegenstrom getrocknet wurde.
Der resultierende Zinkkomplex war analytisch rein.

| | |
|---|---|
| Ausbeute: | 4,55 g (89 % d. Theorie) |
| Schmelzpunkt: | 123°C |

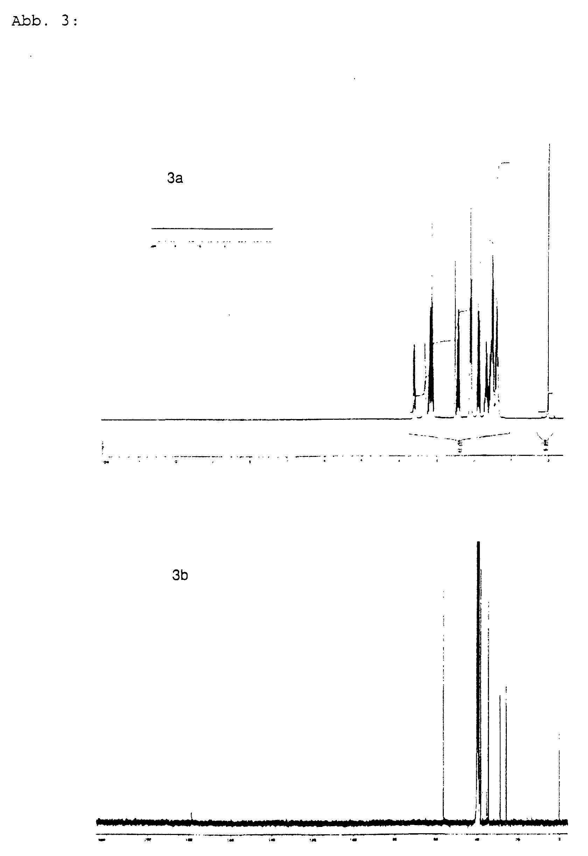

$^1$H-NMR/$^{13}$C-NMR siehe Tabelle 2, Tabelle 3 und Abb.3a ($^1$H) und 3b ($^{13}$C)
IR(KBr) siehe Abb.2

Beispiel 2

Synthese von Zn((rac)-α-Lipoat)$_2$($H_2O$)$_2$

Die Synthese erfolgte analog zu Beispiel 1 unter Verwendung von racemischer α-Liponsäure. Gelbe Nadeln.

| | |
|---|---|
| Ausbeute: | 92 % d. Theorie) |
| Schmelzpunkt: | 112°C |

$^1$H-NMR/$^{13}$C-NMR siehe Tabelle 2, Tabelle 3
IR(KBr) siehe Abb. 1

Die Herstellung der Zn-Komplexe aus Beispiel 1 und 2 erfolgte auch ausgehend von Zinkacetat., -sulfat und -chlorid, und lieferte die Verbindungen aus Beispiel 1 und 2 in vergleichbaren Ausbeuten und Reinheiten.

Tabelle 1

$^{13}$C-NMR-Spektren (D$^6$-DMSO, Angaben in [ppm]) zu Beispiel 1 und 2

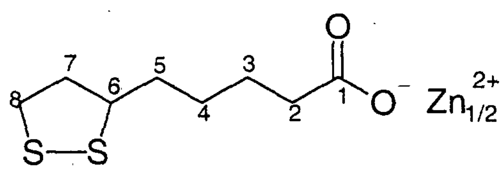

| C-Atom Nr. | Zn((rac)-α-Lipoat)$_2$($H_2O$)$_2$ (Beispiel 2) | Zn((R)-α-Lipoat)$_2$($H_2O$)$_2$ (Beispiel 1) |
|---|---|---|
| 1 | 178 | 178 |
| 2 | 39 | 39 |
| 3 | 25 | 25 |
| 4 | 28 | 28 |
| 5 | 37 | 35 |

(fortgesetzt)

| C-Atom Nr. | Zn((rac)-α-Lipoat)$_2$(H$_2$O)$_2$ (Beispiel 2) | Zn((R)-α-Lipoat)$_2$(H$_2$O)$_2$ (Beispiel 1) |
|---|---|---|
| 6 | 56 | 56 |
| 7 | 34 | 34 |
| 8 | 40 | 40 |

Tabelle 2

$^1$H-NMR-Spektren ($D^6$-DMSO, Angaben in [ppm]) zu Beispiel 1 und 2

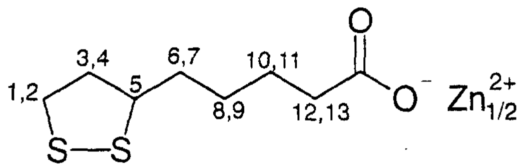

| H-Atom Nr. | Zn ((rac)-α-Lipoat)$_2$(H$_2$O)$_2$ (Beispiel 2) | Zn((R)-α-Lipoat)$_2$(H$_2$O)$_2$ (Beispiel 1) |
|---|---|---|
| 1,2 | 3,15 (m) | 3,15 (m) |
| 3,4 | 2,45 (m) | 2,45 (m) |
| 5 | 3,65 (m) | 3,65 (m) |
| 6 - 11 | 1 - 2 | 1 - 2 |
| 12,13 | 2,05 (t) | 2,05 (t) |

Beispiel 3

Herstellung von weiteren Metall-(R)-α-Lipoaten

Die Synthese erfolgte analog zu Beispiel 1 unter Verwendung von verschiedenen Mineralsalzen. Die Ergebnisse sind in der Tabelle 3 zusammengefaßt.

Tabelle 3

| Beispiel | eingesetztes Mineralsalz | Farbe des Metall-(R)-α-Lipoats | Ausbeute des Metall-(R)-α-Lipoats |
|---|---|---|---|
| 3.1 | $Fe(NO_3)_3$ | hellbraun | 48 % |
| 3.2 | $Cu(NO_3)_2$ | türkis | 84 % |
| 3.3 | $Cr(NO_3)_3$ | blau | 59 % |
| 3.4 | $MnCl_2$ | beige | 60 % |
| 3.5 | $Co(NO_3)_2$ | violett | 45 % |
| 3.6 | $Ca(OH)_2$ | weiß | 68 % |

II. Biologische Beispiele

Bioverfügbarkeitsuntersuchungen von Zn((R)-α-Lipoat)$_2$(H$_2$O)$_2$ in Ratten im Vergleich mit der Bioverfügbarkeit von Zinksulfat

Allgemeine Bedingungen

Zink wird im allgemeinen aus Lebensmitteln tierischer Herkunft besser verwertet als aus pflanzlichen Produkten. Als Hauptursache hierfür wird der höhere Phytinsäuregehalt (PA-Gehalt) in pflanzlichen Produkten angesehen. Zink ist das Spurenelement, dessen Bioverfügbarkeit bei hoher PA-Aufnahme am deutlichsten absinkt. Durch Phytinsäure (PA) wird nicht allein die Bioverfügbarkeit des Nahrungszinks herabgesetzt, sondern auch in hohem Maße endogen sezerniertes Zn der Rückresorption entzogen. Ab einem molaren PA:Zn-Quotienten > 10 bis 15 in der Diät ist unter kontrollierten Bedingungen bei der Ratte mit einer reduzierten Zn-Bioverfügbarkeit zu rechnen.

Um diesen Effekt des natürlichen Phytinsäuregehalts in Futter-oder Nahrungsmitteln zu berücksichtigen, wurden in den Versuchsdiäten, neben einer PA freien Diät auch Diäten mit supplementierter PA verwendet.

Unter NaPA wird im folgenden Natriumphytat, unter AAS Atomabsorptionsspektrometrie, unter AM Anfangsmasse, unter FM Frischmasse, unter HPLC High Performance Liquid Chromatography, unter ICP-AESInductively Coupled Plasma Atomic Emission Spectrometry, unter LM Lebendmasse, unter MT Metallothionein, unter n.n. nicht nachweisbar und unter PE Polyethylen verstanden.

Beispiel 4

Studie 1: Bioverfügbarkeitsuntersuchungen von $Zn((R)\text{-}\alpha\text{-Lipoat})_2(H_2O)_2$ in Ratten

4.1 Herkunft, Haltung der Versuchstiere und Durchführung der Stoffwechselversuche

Es werden männlichen Albinoratten (Wistar) mit einem Anfangsgewicht von 40 g eingesetzt. Die Tiere werden während des jeweils 28 tägigen Versuchszeitraumes bei einer Raumtemperatur von 22°C, einer relativen Luftfeuchte von ca. 55 % sowie einem 12stündigen Hell-Dunkel-Rhythmus gehalten. Die Haltung der Ratten erfolgt einzeln in Makrolonkäfigen mit Edelstahlböden, die eine kontrollierte Futteraufnahme sowie eine quantitative Kot- und Harnsammlung ermöglichten. Die Futterzuteilung erfolgt einmal täglich um 8°°. Die Lebendmasse der Ratten wird wöchentlich ermittelt, Kot- und Harnsammlung erfolgt täglich um 10°° und um 17°°. Zu Versuchsende werden die Ratten nach Chloroformbetäubung dekapitiert.

4.2 Beschreibung der Diäten und Versuchsplan

Um definierte Mineralsalz- und PA-Konzentrationen zu erzielen, wurde eine Versuchsdiät auf der Basis hochreiner Einzelkomponenten erstellt (Tabelle 4), mit der das Zink-Lipoat $Zn((R)\text{-}\alpha\text{-Lipoat})_2(H_2O)_2$ aus Besiepiel 1 und PA verfüttert wurde.

Die Mineralstoff-, Spurenelement- und Vitaminergänzungen erfolgten in Anlehnung an die Empfehlung des NRC (1978). Der native Vitamingehalt der Einzelkomponenten blieb unberücksichtigt. Um etwaigen Aminosäureinbalancen infolge des hohen Gehaltes an schwefelhaltigen Aminosäuren im Eiklarprotein vorzubeugen, wurde synthetisches L-Lysin supplementiert.

Tabelle 4

| Komponente | g/kg |
|---|---|
| Maisstärke | 477 |
| Eiklarprotein | 200 |
| Saccharose | 100 |
| Sojaöl | 70 |
| Cellulose | 30 |
| L-Lysin-HCl | 3 |
| Mineralstoffvormischung[1]) | 100 |
| Vitaminvormischung[2]) | 20 |

[1]) Mineralstoffvormischung (Angaben je kg Diät) 26,73 g $CaHPO_4$ x 2 $H_2O$ reinst; 6,68 $KH_2PO_4$ reinst; 5,07 g; $MgSO_4$ x 7 $H_2O$ reinst; 4,43 g $CaCO_3$ reinst; 2,47 g NaCl reinst; 1,22 g $Na_2CO_3$ reinst; 298,68 mg $FeSO_4$ x 7 $H_2O$ p.A.; 184,59 mg $MnSo_4$ x $H_2O$ p.A.; 87,99 mg $ZnSO_4$ x 7 $H_2O$ p.A.; 27,50 mg $CuSO_4$ x 5 $H_2O$ p.A.; 4,41 mg $Cr(CH_3COO)_3$ p.A.; 2,38 mg $CoSO_4$ x 7 $H_2O$ p.A.; 2,21 mg NaF p.A.; 0,83 mg $Na_2SeO_3$ x 5 $H_2O$ p.A.; 0,65 mg KI p.A.; 0,25 mg $Na_2MoO_4$ x 2 $H_2O$ p.A.

[2]) Vitaminvormischung (Angaben je kg Diät) 1,8 mg Retinol; 0,025 mg Cholecalciterol; 100 mg D,L-α-Tocopherylacetat; 5 mg Menadion; 30 mg Ascorbinsäure; 8 mg Thiaminmononitrat; 10 mg Riboflavin; 10 mg Pyridoxin; 40 mg Niacin; 30 mg Ca-D-Pantothenat; 3 mg Folsäure; 10 mg p-Aminobenzoesäure; 0,2 mg Biotin; 0,05 mg Cobalamin; 100 mg myo-Inositol; 1150 mg Cholinchlorid

Das Mischen der Versuchsdiäten erfolgt in einem Präzisionsmischer aus Edelstahl. Die Vitamin-, Spurenelement- und Phytasevormischungen werden in einem Labormischer erstellt, wobei jeweils Maisstärke als Trägerstoff eingesetzt wird.

Die Lagerung der Diäten erfolgt bei +4°C.

In Tabelle 5 ist der Versuchsplan dargestellt. Je Versuchsgruppe werden 6 Ratten verwendet. Die Zuteilung der mehligen Diäten erfolgt ad libitum. Die Menge an zugegebenem Zink in Form des Zink-lipoats Zn((R)-$\alpha$-Lipoat)-$_2$ $(H_2O)_2$ beträgt 10 mg/kg Futter (Gruppe 1 und 2) und 20 mg/kg Futter (Gruppe 3). Durch teilweisen Austausch der Maisstärke durch 0,4 Gew.-% in Form von NaPA werden molare PA:Zn-Quotienten von 19,8:1 bzw. 39,6:1 eingestellt. Die Kontrollgruppe (Gruppe 1) erhielt die PA-freie Basisdiät.

Tabelle 5

| Gruppe | Anzahl Tiere [n] | Menge an Zn als Zn-Lipoat [mg/kg] Diät | PA (Gew.-%) | PA:Zn (molar) |
|---|---|---|---|---|
| 1 | 6 | 10 | - | - |
| 2 | 6 | 10 | 0,4 | 39,6 : 1 |
| 3 | 6 | 20 | 0,4 | 19,8 : 1 |

4.3 Messen der Bioverfügbarkeitsparameter - Gewinnung und Aufbereitung des Analysenmaterials

4.3.1 Kot- und Harnproben

Die getrennt in den Stoffwechselkäfigen anfallenden Kot-und Harnmengen werden täglich quantitativ gesammelt und bei -22°C gelagert. Zur Vermeidung etwaiger N-Verluste in den Harnauffangbehältern wird täglich jeweils 1 mL 20%ige HCl (suprapur) vorgelegt. Zur weiteren Analyse wird das Probenmaterial für 48 h gefriergetrocknet.

4.3.2 Organ- und Gewebeproben

Der rechte Femur, Leber und Testes werden nach Dekapitation und vollständigem Entbluten der Tiere sofort entnommen und die Frischmasse ermittelt. Bis zur weiteren Analyse werden die Proben in Kunststoff-Folie eingeschweißt und bei -22°C gelagert.

4.3.3 Blut

Die Ratten werden bei Versuchsende nach Vollnarkose mit Chloroform dekapitiert und das Blut in heparinisierten Kunststoffröhrchen aufgefangen. Pro mL Blut werden 40 I.E. Heparin-Natrium vorgelegt.

Unmittelbar nach der Entnahme werden die Blutproben für 15 Minuten bei 16000 g zentrifugiert und das Plasma in 2-ml-Reaktionsgefäßen pipettiert. Die Proben wurden bis zur weiteren Analyse bei -80°C gelagert.

4.4 Herstellung der Aschelösungen und Zn-Bestimmung

Die Diät-, Kot-, Harn- und Gewebeproben werden ausschließlich trocken verascht. Die eingesetzten Glaswaren werden vor der Verwendung mit 20%iger $HNO_3$ (p.A.) gereinigt und mehrfach mit bidestilliertem Wasser gespült. Die Quarztiegel werden über Nacht bei 750°C im Muffelofen vorgeglüht.

Die Trockenveraschung erfolgt über 18 h bei 450°C.

Nach der Veraschung werden die Proben mit 3M HCL (suprapur) versetzt, mit einem Uhrglas abgedeckt und für 10 Minuten auf einem siedenden Wasserbad erhitzt. Die Volumina des Säurezusatzes werden nach der Menge der herzustellenden Aschelösungen bemessen, die jeweils in einer 0,3 M Endkonzentration vorliegen sollten. Nach dem Abkühlen werden die Proben mit heißem aqua bidest, durch ein aschefreies Rundfilter in einem Schliffmeßkolben filtriert. Unvollständig veraschte Proben werden bis zur weiteren Analyse in Polyäthylenflaschen aufbewahrt.

Zink wird mittels Atomabsorptionsspektrophotometrie (AAS) in einer Acetylen-Flamme gemessen.

4.5 Diagnose des Zink-Status

4.5.1 Bestimmung der Plasma-Zink-Konzentration

Die Plasma-Zink-Konzentration wird mittels AAS (Philips, PIJ 9400) direkt in der Flamme gemessen. Die Proben werden mit 0,1 M HCl im Verhältnis 1:20 (v/v) verdünnt.

4.5.2 Bestimmung der freien Zink-Bindungskapazität

Die prozentuale freie Zn-Bindungskapazität wird nach der Methode von KINCAID und CRONRATH (J. Dairy Sci. 1979, 62, 120, 1474-1479) in der Modifikation von ROTH und KIRCHGESS-NER (Res. Exp. Med. 1980, 177, 213-219) durchgeführt. Um das limitierte Plasmavolumen effektiv einzusetzen, werden die einzelnen Arbeitsschritte wie folgt verändert: 0,4 mL Plasma werden mit dem gleichen Volumen einer $ZnCl_2$-Lösung (5 µg Zn/mL) versetzt (Absättigung freier Zn-Bindungsstellen der Plasmaproteine). Zur Fällung des überschüssigen, nicht an Proteine gebundenen Zinks werden 40 mg basisches Magnesiumcarbonat zugegeben. Nach Zentrifugation der Proben werden 0,4 mL des im Überstand befindlichen Zn-gesättigten Plasmas abpipettiert und mit 0,6 mL des im Überstand befindlichen Zn-gesättigten Plasmas abpipettiert und mit 0,6 mL 0,1 M HCl versetzt. Die Zn-Bestimmung erfolgt mittels AAS direkt in der Flamme. Die freie Zn-Bindungskapazität errechnet sich aus der Differenz zwischen dem Zn-Gehalt des abgesättigten und des unbehandelten Plasmas, wobei als Bezugsgröße die Zn-Konzentration des abgesättigten Plasmas zugrunde gelegt wird.

4.5.3 Bestimmung der Aktivität der Alkalischen Phosphatase

Die Aktivität des Zn-Metalloenzyms Alkalische Phosphatase (E.C. 3.1.3.1) wird unter Verwendung der Einzelchemikalien Diethanolaminpuffer und p-Nitrophenylphosphat nach den Empfehlungen der DEUTSCHEN GESELLSCHAFT FÜR KLINISCHE CHEMIE (Z. Klin. Chem. u. klin. Biochem. 1972, 10, 191) durchgeführt:

Die AP Katalysiert folgende Reaktion:

$$\text{p-Nitrophenylphosphat} + H_2O \xrightarrow{AP} \text{p-Nitrophenol} + Pi$$

Die enzymkinetische Messung wird im Halbmikromaßstab bei einer Wellenlänge von 405 nm und 25°C am UV-Spektralphotometer durchgeführt. Bei allen Analysenreihen wird ein Kontrollserum mit Werten im physiologischen Normalbereich mitgeführt.

Vergleichsbeispiel 1

Studie 2: Bioverfügbarkeit von $ZnSO_4$ in Ratten

Die Durchführung und Auswertung der Versuche erfolgt analog zu Beispiel 4 unter Verwendung von $ZnSO_4$ als Zn-Quelle. In Tabelle 6 ist der Versuchsplan dargestellt. Je Versuchsgruppe werden 6 Ratten verwendet. Die Zuteilung der mehligen Diäten erfolgt ad libitum. Die Menge an zugegebenem Zink in Form Zinksulfat beträgt 10mg/kg Futter (Gruppe 4 und 5) und 20 mg/kg Futter (Gruppe 6). Durch teilweisen Austausch der Maisstärke durch 0,4 Gew.-% in Form von NaPA werden molare PA:Zn-Quotienten von 19,8:1 bzw. 39,6:1 eingestellt. Die Kontrollgruppe (Gruppe 1) erhielt die PA-freie Basisdiät.

Tabelle 6

| Gruppe | Anzahl Tiere [n] | Menge an Zn als $ZnSO_4$ [mg/kg]Diät | PA (Gew.-%) | PA:Zn (molar) |
|---|---|---|---|---|
| 4 | 6 | 10 | - | - |
| 5 | 6 | 10 | 0,4 | 39,6 : 1 |
| 6 | 6 | 20 | 0,4 | 19,8 : 1 |

Auswertung der Ergebnisse aus Beispiel 4 (Studie 1) und Vergleichsbeispiel 1 (Studie 2)

Die Zulage des NaPA führt zu typischen Zn-Mangelerscheinungen wie Anorexie, Alopezie und einer Depression des Wachstums. Diese Effekte sind bei Zulage von 20 mg Zn/kg Futter schwächer ausgeprägt als bei einer Dosierung von 10 mg/kg.

Im direkten Vergleich von Zn-Lipoat (Beispiel 4) und Zinksulfat (Vergleichsbeispiel 1) zeigt sich eine deutliche Überlegenheit des Lipoats, da die Mangelerscheinungen beim Sulfat noch vorhanden sind, wohingegen diese bei Zink-Lipoat in der hohen Dosierung vollständig verschwinden.

Die scheinbare Absorption und Retention von Zn aus Zink-Lipoat ist bei beiden Dosierungen gesteigert.

Ebenso wie die scheinbare Zn-Absorption und -Retention werden verschiedene Zn-Statusparameter des Blutplasmas (Zn-Konzentration, freie Zink-Bindungskapazität, Alkalische Phosphatase Aktivität) und die Femur- sowie Leber-Zn-Konzentration durch Zn-Lipoat im Vergleich zu Zn-Sulfat verbessert.

Insgesamt läßt sich feststellen, daß Zink-Lipoate fast alle Parameter die zur Diagnose des Zn-Status geeignet sind, im Vergleich zum Zink-Sulfat positiv beeinflußt, was eine erhöhte Bioverfügbarkeit dokumentiert.

Beispiel 5 und Vergleichsbeispiel 2

Studie 3: Bioverfügbarkeitsuntersuchungen von Zn((R)-$\alpha$-Lipoat)$_2$-($H_2O$)$_2$ in Ratten im Vergleich mit der Bioverfügbarkeit von Zink-sulfat

5.1 Versuchsplan

36 männliche Wistar-Albinoratten (Harlan Winkelmann, Borchen) mit einem Ausgangsgewicht von 47,0 ± 2,63 g wurden randomisiert in 6 Gruppen zu jeweils 6 Tieren eingeteilt und über einen Zeitraum von 28 d mit den in Tabelle 7 (Versuchsplan) und Abschnitt 5.2 angeführten Diäten gefüttert. Die Haltung der Tiere erfolgte einzeln in Makrolonkäfigen auf Edelstahlrosten unter Standardbedingungen (22°C, 55 % Luftfeuchte, 12-stündiger Hell-Dunkel-Rhythmus). Futter sowie entionisiertes Trinkwasser standen den Versuchstieren ad *libitum* zur Verfügung. Während der 2. und 3. Versuchswoche (*Stoffwechselphase*) erfolgte eine quantitative Kotsammlung. Nach Abschluss der Versuchsphase (Tag 28) wurden die Tiere unter $CO_2$-Betäubung dekapitiert, entblutet und die zu analysierenden Gewebe entnommen.

Die Gruppen Ib, IIb und IIIb stellen dabei Beispiel 5 dar, während die GruppenIa, IIa und IIIa das Vergleichsbeispiel 2 darstellen.

Tabelle 7:

| Gruppe | Tiere (n) | Zn (mg/kg) | Zn-Verbindung | Phytinsäure (0,4 % als NaPA) |
|---|---|---|---|---|
| Ia | 6 | 10 | Zn-Sulfat | - |
| Ib | 6 | 10 | Zn-Lipoat | - |
| IIa | 6 | 10 | Zn-Sulfat | + |
| IIb | 6 | 10 | Zn-Lipoat | + |
| IIIa | 6 | 20 | Zn-Sulfat | + |
| IIIb | 6 | 20 | Zn-Lipoat | + |
| | Σ=36 | | | |

Als Parameter zur Einschätzung der Zn-Verfügbarkeit wurden die zootechnischen Merkmale Futteraufnahme, Lebendmasseentwicklung und Lebendmassezuwachs, die scheinbare Zn-Absorption, die Aktivität der Alkalischen Phosphatase im Plasma, die Plasma- und Femur-Zn-Konzentration sowie die Metallothionein-Konzentration im Lebergewebe herangezogen.

5.2 Versuchsdiäten

Die Versuchstiere wurden mit halbsynthetischen Diäten auf Basis hochreiner Komponenten (Maisstärke, Eiklarprotein, Saccharose, Sojaöl und Cellulose) über einen Zeitraum von 28 Tagen gefüttert (Zusammensetzung der Basisdiät: Tabelle 8), wobei sich die Versuchsdiäten hinsichtlich der Art der supplementierten Zinkverbindung, der absoluten Zn-Konzentration sowie dem Phytinsäuregehalt (0,4 % PA aus NaPA, Sigma-Aldrich, Steinheim) unterschieden (siehe Tabelle 7).

Als Zn-Supplemente wurden Zn((R)-$\alpha$-Lipoat)$_2$($H_2O$)$_2$ aus Beispiel 1 (Beispiel 5) sowie Zn-Sulfat ($ZnSO_4$ x 7 $H_2O$ p.a., Merck, Darmstadt) (Vergleichsbeispiel 2) eingesetzt. Die Zn-Konzentrationen der Versuchsdiäten Ia - IIb wurden auf eine für wachsende Ratten als marginal einzuschätzende Höhe von 10 mg/kg Diät eingestellt, um eine Überprüfung der Verfügbarkeit der zu untersuchenden Zn-Supplemente im suboptimalen Versorgungsbereich zu ermöglichen. Die gegenwärtigen Zufuhrempfehlungen des NATIONAL RESEARCH COUNCIL für wachsende Laborratten betragen 12 mg Zn je kg Diät (NRC 1995), sofern die Diät kein Phytat enthält.

Tabelle 8

| Komponente | g/kg |
|---|---|
| Maisstärke | 477 |
| Eiklarprotein | 200 |
| Saccharose | 100 |
| Sojaöl | 70 |
| Cellulose | 30 |
| L-Lysin-HCl | 3 |
| Mineralstoffvormischung [1)] | 100 |
| Vitaminvormischung [2)] | 20 |
| | 1000 |

[1)] Mineralstoffvormischung (Angaben je kg Diät): 16,17 g $CaHPO_4$ x 2 $H_2O$; 8,91 g $K_2HPO_4$; 8,08 g $CaCO_3$; 6,08 g $MgSO_4$ x 7 $H_2O$; 1,16 g NaCl; 0,58 g $Na_2CO_3$; 298,68 mg $FeSO_4$ x 7 $H_2O$; 61,52 mg $MnSO_4$ x $H_2O$; 31,45 mg $CuSO_4$ x 5 $H_2O$; 9,61 mg $KCr(SO_4)_2$ x 12 $H_2O$; 2,38 mg $CoSO_4$ x 7 $H_2O$; 2,21 mg NaF; 0,83 $Na_2SeO_3$ x 5 $H_2O$; 0,52 mg KI; 0,50 mg $NaMoO_4$ x 2 $H_2O$

[2)] Vitaminvormischung (Angaben je kg Diät): 4500 IE Vitamin A; 1100 IE Vitamin $D_3$; 80 IE Vitamin E; 0,9 mg Menadion; 30 mg Ascorbinsäure; 6 mg Thiamin; 7 mg Riboflavin; 7 mg Pyridoxin; 0,03 mg Cobalamin; 0,25 mg Biotin; 2,5 mg Folsäure; 35 mg Nicotinsäure; 20 mg Pantothensäure; 1100 mg Cholinchlorid; 100 mg Inosit; 10 mg p-Aminobenzoesäure

Mit Ausnahme der angeführten Zn-Stufe von 10 mg/kg Diät lagen die angestrebten Diätkonzentrationen von Mineralstoffen (Tabelle 9: Angestrebte Mengen- und Spurenelementkonzentrationen der Versuchsdiäten sowie Empfehlungen des NRC für wachsende Laborratten ) und von Vitaminen stets deutlich höher als die Empfehlungen des NATIONAL RESEARCH COUNCIL für die wachsende Laborratte (NRC 1995) und sind somit als sicher bedarfsdeckend einzuschätzen.

Tabelle 9 zeigt die angestrebte Mengen- und Spurenelementkonzentrationen der Versuchsdiäten sowie Empfehlungen des NRC für wachsende Laborratten (NRC 1995)

Tabelle 9:

| Mengenelemente | angestrebt (g/kg) | *NRC (g/kg)* | Spurenelemente | angestrebt mg/kg | *NRC (mg/kg)* |
|---|---|---|---|---|---|
| Calcium | 7,0 | *5,0* | Eisen | 60 | *35* |
| Phosphor | 4,5 | *3,0* | Mangan | 20 | *10* |
| Magnesium | 0,6 | *0,5* | Zink | 10/20 | *12* |

5.3 Analytische Methoden

5.3.1 Analyse der Diäten

Die Bestimmung der Trockenmasse sowie der Rohnährstoffe (Rohprotein, Rohfett, Rohfaser, Rohasche) erfolgte nach den Vorschriften des VDLUFA-Methodenbuches zur chemischen Untersuchung von Futtermitteln (NAUMANN, C.; BASSLER, R. (1997): Methodenbuch Band III. Die chemische Untersuchung von Futtermitteln, 4. Ergänzungslieferung. VDLUFA-Verlag, Darmstadt). Die Bruttoenergie der Versuchsdiäten wurde unter Einsatz eines adiabatischen Bombenkalorimeters (IKA-Kalorimeter C400, Jahnke und Kunkel, Staufen) ermittelt.

Die quantitative Bestimmung der Phytinsäurekonzentration in den Versuchsdiäten erfolgte mittels HPLC nach der Methode von NEUSSER und PALLAUF (NEUSSER, H.; PALLAUF, J. (1988) : Bestimmung von Phytinsäure in Futtermitteln und Faeces mittels Hochdruckfüssigkeits-Chromatographie. J. Anim. Physiol. a. Anim. Nutr. **60,** 20.).

Die Veraschung der Diätproben geschah nach dem Prinzip der Trockenveraschung. Hierbei wurde die organische Matrix thermisch zersetzt und vollständig mineralisiert. Die Anzahl der veraschten Parallelen je Diät betrug n = 2. Der Ascherückstand wurde durch Säurezugabe in Lösung gebracht und anschließend quantitativ analysiert. Die Ca-, Mg-, P-, Fe- und Mn-Konzentrationen der Diäten wurden mittels ICP-AES (PU 701, Unicam, Kassel), die Zn-Konzentration mittels Flammen-AAS (Philips, PU 9400, Kassel) analysiert. Die Validierung der Analysen erfolgte durch Standard-Addition.

5.3.2 Zn-Konzentration im Femur

Die Herstellung der Aschelösungen erfolgte nach dem Prinzip der Nassveraschung. Das Probenmaterial wurde mit 10 ml 65%iger $HNO_3$ (Merck, suprapur) versetzt und anschließend unter Rückflusskühlung in einer Aufschlussapparatur (Gerhardt SMA-20, Bonn) gekocht. Hierbei wurde die organische Matrix oxidativ abgebaut und das Untersuchungsmaterial vollständig mineralisiert. Die Zn-Konzentration in den Aschelösungen wurde mittels Flammen-AAS (Philips, PU 9400, Kassel) bestimmt.

5.3.3 Analyse der Kotproben

Während der zweiwöchigen Stoffwechselphase wurde der Rattenkot täglich gesammelt, anhaftende Futterreste und andere Verunreinigungen entfernt und die Proben bei -20°C gelagert. Am Ende der Stoffwechselperiode wurde die Kotmenge quantitativ erfasst und anschließend unter Vakuum über einen Zeitraum von 48 h gefriergetrocknet (Gamma 1-20, Christ, Osterode). Die getrockneten Proben wurden mit einer haushaltsüblichen Mühle (Moulinette electronic 89902) feingemahlen und in PE-Flaschen unter Umgebungstemperatur gelagert.

Ein Probenaliquot von 1,5 g der gefriergetrockneten und gemahlenen Kotproben wurde über 24 h bei 105°C zur Entfernung des Restwassers getrocknet und anschließend im Muffelofen über 24 h bei 450°C verascht. Der Ascherückstand wurde durch Säurezugabe in Lösung gebracht und anschließend quantitativ analysiert. Die Zn-Konzentration in den Kotproben wurde mittels Flammen-AAS (Philips, PU 9400, Kassel) bestimmt.

5.3.4 Diagnose des Zn-Status

Die Zn-Absorption wurde ohne Berücksichtigung der endogenen Quote als Differenz aus Elementaufnahme und faecaler Exkretion berechnet (= *scheinbare Absorption*). Die Bestimmung der Plasma-Zn-Konzentration erfolgte mittels AAS (Philips, PU 9400, Kassel). Die Proben wurden mit 0,1 M HCl im Verhältnis 1 : 20 (v/v) verdünnt. Die Aktivität des Zn-Metalloenzyms Alkalische Phosphatase (AP) (E.C. 3.1.3.1) wurde unter Verwendung der Einzelchemikalien Diethanolaminpuffer und p-Nitrophenylphosphat nach den Empfehlungen der DEUTSCHEN GESELLSCHAFT FÜR KLINISCHE CHEMIE (DEUTSCHE GESELLSCHAFT FÜR KLINISCHE CHEMIE (1972) : Standardmethode zur Bestimmung der Aktivität der alkalischen Phosphatase (AP). Z. klin. Chem. u. klin. Biochemie. **10**, 191.) durchgeführt.

5.3.5 Metallothionein im Lebergewebe

Die Bestimmung der Gesamt-MT-Konzentration im Lebergewebe erfolgte anhand der Cadmium-Bindungsmethode nach EATON und TOAL (EATON, D. L.; TOAL, B. F. (1982) : Evaluation of the cadmium/hemoglobin affinity assay for the rapid determination of metallothionein in biological tissues. Toxicol. Appl. Pharmacol. **66,** 134-142.) in einer am Giessener Institut bewährten Modifikation (LEHNERT, V. (1994): Einfluß von Zink sowie fetalem Kälberserum, bovinem Serumalbumin und calciummobilisierenden Rezeptoragonisten auf Metallothionein in primär kultivierten Rattenhepatocyten. Inaugural-Dissertation am Institut für Tierernährung und Ernährungs-physiologie der Universität Giessen, Verlag Shaker, Aachen., PALLAUF et al. 1995). Der Proteingehalt in den Leberhomogenaten wurde mit der Lowry-Folin-Methode bestimmt (DAWSON, R.M. C.; ELLIOTT, D.C.; JONES, K.M. (1986): Data for biochemical research, 3$^{rd}$ ed., Clarendon Press, New York, 543).

5.3.5 Statistische Auswertung

Die deskriptive Aufbereitung und graphische Darstellung des Datenmaterials wurde mit Hilfe des Tabellenkalkulationsprogramms Microsoft Excel 2000 durchgeführt. Die statistische Auswertung der Versuchsergebnisse erfolgte mittels SPSS (Statistical Package for the Social Sciences) für Windows (Version 10.0) und umfasste die Prüfung auf Normalverteilung (KOLMOGOROV-SMIRNOW- und SHAPIRO-WILKS-Test) und Varianzhomogenität (LEVENE-Test) sowie die einfaktorielle Varianzanalyse (Prozedur ONEWAY) mit anschließendem Test auf die Signifikanz von Mittelwertunterschieden nach TUKEY-HSD. Das Signifikanzniveau wurde auf 5 % ($p < 0,05$) festgelegt. Bei nichtgegebener Varianzhomogenität wurde die Signifikanz von Mittelwertunterschieden anhand des GAMES HOWELL-Tests überprüft.

Die in den Tabellen aufgeführten Ergebnisse stellen den Gruppenmittelwert (M) sowie die Standardabweichung (SD) der Einzelwerte dar. Signifikante Mittelwertsunterschiede wurden durch unterschiedliche Hochbuchstaben gekennzeichnet.

5.4. Ergebnisse

5.4.1 Versuchsdiäten

Die analytisch ermittelten Gehalte an Rohnährstoffen sowie die Bruttoenergie der Versuchsdiäten sind in

Tabelle 10 aufgeführt. Alle Diäten wiesen innerhalb der üblichen Schwankungsbreiten vergleichbare Trockenmassen-, Rohprotein-, Rohfett-, Rohfaser- und Bruttoenergiegehalte auf. Der Anstieg des Rohaschegehaltes in den Diäten IIa - IIIb ist auf den Phosphor-Eintrag infolge der PA-Supplementation zurückzuführen.

Tabelle 10:

| Versuchsdiäten Ia - IIIb | | | |
|---|---|---|---|
| Trockenmasse | (% der AM) | 91,6 | ± 0,13 |
| Rohprotein | (% der AM) | 16,7 | ± 0,06 |
| Rohfett | (% der AM) | 7,16 | ± 0,02 |
| Rohfaser | (% der AM) | 5,21 | ± 0,48 |
| Rohasche (Diäten Ia, Ib) | (% der AM) | 4,31 | ± 0,02 |
| Rohasche (Diäten IIa - IIIb) | (% der AM) | 4,85 | ± 0,07 |
| Bruttoenergie | (kJ/g AM) | 17,8 | ± 0,19 |

Die Mineralstoffkonzentrationen der Versuchsdiäten sind in Tabelle 11 dargestellt. Die Elementkonzentrationen waren innerhalb der Analysenschwankungen konform. Es konnte eine hinreichende Übereinstimmung mit den angestrebten Werten erzielt werden. Höhere Phosphor-Gehalte in den Diäten IIa - IIIb waren auf die PA-Zulagen von 0,4 % zurückzuführen.

Tabelle 11

| Diät | Ca (g/kg) | Mg (g/kg) | P (g/kg) | Fe (mg/kg) | Zn (mg/kg) | Mn (mg/kg) |
|---|---|---|---|---|---|---|
| Ia | 6,93 | 0,75 | 4,78 | 53,7 | 12,1 | 21,2 |
| Ib | 6,99 | 0,89 | 4,81 | 71,2 | 10,2 | 18,4 |
| IIa | 7,20 | 0,92 | 5,92 | 69,2 | 10,4 | 19,9 |
| IIb | 7,40 | 0,91 | 6,04 | 67,9 | 9,93 | 20,9 |
| IIIa | 7,44 | 0,81 | 6,20 | 78,2 | 19,9 | 20,0 |
| IIIb | 7,09 | 0,92 | 6,07 | 62,8 | 21,1 | 16,2 |

Die analysierten Phytinsäuregehalte, molaren PA : Zn- sowie PA x Ca : Zn-Quotienten der Versuchsdiäten sind in Tabelle 12 dargestellt. Die PA-Konzentrationen entsprachen den Erwartungswerten und waren innerhalb der Analysenschwankungen konform.

Tabelle 12

| Diät | PA (%) | PA : Zn (molar) | PA x Ca : Zn (molar) |
|---|---|---|---|
| Ia | n.n. | - | - |
| Ib | n.n. | - | - |
| Iia | 0,40 | 38,7 | 6,96 |
| Iib | 0,39 | 38,4 | 7,11 |
| IIIa | 0,41 | 20,4 | 3,79 |
| IIIb | 0,40 | 18,9 | 3,35 |

5.4.2 Zootechnische Parameter

Die mittleren wöchentlichen Futteraufnahmen sowie die Gesamtfutteraufnahme im 28-tägigen Versuchszeitraum sind in Tabelle 13 aufgeführt. Bei Verfütterung PA-haltiger Diäten mit niedriger Zn-Stufe (Gruppe IIa und IIb) wurde eine signifikant geringere Gesamtfutteraufnahme im Vergleich zu den Gruppen Ia und Ib sowie IIIa und IIIb festgestellt. Es konnte eine Steigerung infolge der Zn-Lipoat-Verfütterung beobachtet werden.

Das geringste Wachstum der Ratten war bei Verfütterung PA-haltiger Diäten mit niedriger Zn-Konzentration (Gruppen IIa und IIb) zu beobachten (Tabelle 14). Sowohl die Verfütterung PA-freier Diäten mit niedriger Zn-Stufe (Gruppen Ia und Ib) als auch die Verfütterung PA-haltiger Diäten mit höherer Zn-Konzentration (Gruppen IIIa und IIIb) resultierten in einem signifikant besseren LebendmasseZuwachs. Weiterhin konnte jeweils eine Steigerung des LebendmasseZuwachs durch Zn-Lipoat im Vergleich zu Zn-Sulfat aufgezeigt werden.

Tabelle 13 zeigt die Futteraufnahme je Woche (g) und Gesamtfutteraufnahme (g/28d) wachsender Ratten

bei Zulage von Zn-Sulfat, Zn-Lipoat und Phytinsäure (PA) (n = 6 x 6). Unterschiedliche Hochbuchstaben innerhalb einer Spalte kennzeichnen signifikante Differenzen von mind. $p < 0,05$ (Tukey-HSD), *kursiv* dargestellte unterschiedliche Hochbuchstaben innerhalb einer Spalte kennzeichnen signifikante Differenzen von mind. $p < 0,05$ (Games Howell)

Tabelle 13

| Futteraufnahme | | | | | | |
|---|---|---|---|---|---|---|
| Gruppe | | $d_1$ - $d_7$ | $d_8$ - $d_{14}$ | $d_{15}$ -$d_{21}$ | $d_{22}$ -$d_{28}$ | gesamt |
| Ia, 10 ppm Zn (Sulfat) | M | 40,4 $^{\mathit{ab}}$ | 67,8 $^{bc}$ | 77,5 $^{bc}$ | 86,6 $^{b}$ | 272 $^{b}$ |
| | SD | ±20,9 | ±12,9 | ±12,4 | ±6,93 | ±50,7 |
| Ib, 10 ppm Zn (Lipoat) | M | 54,9 $^{\mathit{ab}}$ | 78,7 $^{c}$ | 86,9 $^{c}$ | 86,5 $^{b}$ | 307 $^{b}$ |
| | SD | ±11,8 | ±5,82 | ±5,27 | ±5,20 | ±17,4 |
| IIa, 10 ppm Zn (Sulfat) + PA | M | 37,4 $^{\mathit{a}}$ | 42,3 $^{a}$ | 40,5 $^{a}$ | 45,3 $^{a}$ | 166 $^{a}$ |
| | SD | ±11,3 | ±8,30 | ±4,40 | ±7,30 | ±25,2 |
| IIb, 10 ppm Zn (Lipoat) + PA | M | 39,5 $^{\mathit{ab}}$ | 46,5 $^{a}$ | 48,2 $^{a}$ | 51,3 $^{a}$ | 186 $^{a}$ |
| | SD | ±14,3 | ±4,49 | ±6,17 | ±4,18 | ±20,8 |
| IIIa, 20 ppm Zn (Sulfat) + PA | M | 49,6 $^{\mathit{ab}}$ | 61,0 $^{b}$ | 67,9 $^{b}$ | 74,8 $^{b}$ | 253 $^{b}$ |
| | SD | ±15,9 | ±7,75 | ±10,2 | ±10,6 | ±38,2 |
| IIIb, 20 ppm Zn (Lipoat) + PA | M | 60,0 $^{\mathit{b}}$ | 77,3 $^{c}$ | 79,2 $^{bc}$ | 83,5 $^{b}$ | 300 $^{b}$ |
| | SD | ±3,86 | ±6,63 | ±8,25 | ±6,76 | ±22,5 |

Tabelle 14 zeigt die Lebendmasseentwicklung und Lebendmassezuwachs wachsender Ratten bei Zulage von Zn-Sulfat, Zn-Lipoat und Phytinsäure (PA) (n = 6 x 6). Unterschiedliche Hochbuchstaben innerhalb einer Spalte kennzeichnen signifikante Differenzen von mind. $p < 0,05$ (Tukey-HSD), *kursiv* dargestellte unterschiedliche Hochbuchstaben innerhalb einer Spalte kennzeichnen signifikante Differenzen von mind. $p < 0,05$ (Games Howell).

Tabelle 14

| | | Lebendmasse (g) | | | | | |
|---|---|---|---|---|---|---|---|
| Gruppe | | $d_0$ | $d_7$ | $d_{14}$ | $d_{21}$ | $d_{28}$ | LM-Zuwachs |
| Ia, 10 ppm Zn (Sulfat) | M | 47,3 | 58,1 $^{\mathit{ab}}$ | 104,2 $^{\mathit{ab}}$ | 141,3 $^{bc}$ | 175,8 $^{bc}$ | 128,5 $^{bc}$ |
| | SD | ±3,38 | ±20,5 | ±21,5 | ±21,5 | ±17,4 | ±20,0 |
| Ib, 10 ppm Zn (Lipoat) | M | 47,1 | 75,6 $^{\mathit{ab}}$ | 122,5 $^{\mathit{b}}$ | 159,2 $^{c}$ | 185,8 $^{c}$ | 138,7 $^{c}$ |
| | SD | ±3,01 | ±8,96 | ±8,36 | ±7,63 | ±7,22 | ±7,68 |
| IIa, 10 ppm Zn (Sulfat) + PA | M | 45,8 | 62,0 $^{\mathit{a}}$ | 84,6 $^{\mathit{a}}$ | 94,2 $^{a}$ | 106,5 $^{a}$ | 60,7 $^{a}$ |
| | SD | ±2,78 | ±5,47 | ±13,2 | ±13,7 | ±15,4 | ±13,4 |
| IIb, 10 ppm Zn (Lipoat) + PA | M | 47,2 | 60,0 $^{\mathit{ab}}$ | 82,5 $^{\mathit{a}}$ | 99,1 $^{a}$ | 114,2 $^{a}$ | 67,0 $^{a}$ |
| | SD | ±2,52 | ±14,3 | ±11,2 | ±10,4 | ±8,39 | ±6,31 |
| IIIa, 20 ppm Zn (Sulfat) + PA | M | 47,2 | 68,4 $^{\mathit{ab}}$ | 102,4 $^{\mathit{ab}}$ | 132,3 $^{b}$ | 161,9 $^{b}$ | 114,7 $^{b}$ |
| | SD | ±2,49 | ±15,9 | ±12,1 | ±14,7 | ±14,9 | ±15,4 |
| IIIb, 20 ppm Zn (Lipoat) + PA | M | 47,3 | 77,9 $^{\mathit{b}}$ | 119,1 $^{\mathit{b}}$ | 147,4 $^{bc}$ | 173,2 $^{bc}$ | 125,9 $^{bc}$ |
| | SD | ±2,36 | ±4,57 | ±9,00 | ±12,3 | ±11,4 | ±10,4 |

5.4.3 Parameter der Zinkverfügbarkeit und des Zinkstatus

Im Verlauf der 14-tägigen Stoffwechselphase lag die Zn-Aufnahme der Gruppen IIa und IIb (10 mg Zn/kg Diät + 0,4 % PA) signifikant niedriger als die Zn-Aufnahme der Gruppen Ia und Ib (Tabelle 15). Bei einer Erhöhung der

diätetischen Zn-Konzentration auf 20 mg je kg Diät (IIIa, IIIb) wurde eine signifikante Steigerung der Zn-Zufuhr um annähernd den Faktor 2 erreicht. Weiterhin ergab sich bei Verfütterung PA-haltiger Diäten mit hoher Zn-Stufe (20 mg/kg Diät) eine signifikant höhere Zn-Aufnahme infolge der Zn-LipoatSupplementation.

Unabhängig von der PA-Supplementation lag die faecale Zn-Exkretion der Gruppen Ia, Ib, IIa und IIb auf einer Höhe von 0,411 - 0,494 mg/14 d (Tabelle 15). Die Zn-Exkretion über den Kot erreichte bei den Gruppen IIIa und IIIb infolge der diätetischen Zn-Konzentration von 20 mg/kg ein signifikant höheres Niveau (1,43 - 1,71 mg/14 d) als in den Gruppen mit niedriger Zn-Stufe.

Die absolute scheinbare Absorption (mg Zn/14 d) lag bei den PA-frei ernährten Ratten (Gruppen Ia und Ib) bei 1,24 bzw. 1,35 mg/14 d (Tabelle 15). Bei Zulage von 0,4 % PA (Gruppen IIa, IIb) wurde eine drastische Reduktion der Zn-Absorption auf Werte von 0,351 bzw. 0,446 mg/14 d ersichtlich. Bei einer Verdopplung der Zn-Stufe auf 20 mg/kg Diät (Gruppen IIIa und IIIb) wurde trotz eines PA-Gehaltes von 0,4 % ein Anstieg der absoluten Zn-Absorption auf Werte von 1,13 bzw. 1,60 mg/14 d ersichtlich. Innerhalb dieser beiden Gruppen ergab sich eine statistisch signifikante höhere absolute Zn-Absorption in der Gruppe mit Zn-Lipoat (1,60 mg/14 d).

Bei den PA-frei ernährten Gruppen Ia und Ib wurde eine hohe scheinbare Zn-Absorption von 73,6 bis 76,4 % erreicht. Nach Zulage von PA lag die prozentuale Zn-Absorption der übrigen Gruppen (IIa bis IIIb) ungeachtet der absoluten Zn-Konzentration mit 43,0 bis 48,4 % auf statistisch vergleichbarem Niveau. Es zeigte sich jedoch eine Steigerung der scheinbaren Zn-Absorption von Gruppe IIa zu Gruppe IIb sowie Gruppe IIIa zu Gruppe IIIb infolge der Zn-Lipoat-Supplementation.

Tabelle 15 zeigt die Aufnahme, faecale Exkretion und scheinbare Absorption von Zink wachsender Ratten bei Zulage von Zn-Sulfat, Zn-Lipoat und Phytinsäure (PA) (n = 6 x 6). Unterschiedliche Hochbuchstaben innerhalb einer Spalte kennzeichnen signifikante Differenzen von mind. $p < 0,05$ (Tukey-HSD), *kursiv* dargestellte unterschiedliche Hochbuchstaben innerhalb einer Spalte kennzeichnen signifikante Differenzen von mind. $p < 0,05$ (Games Howell)

Tabelle 15

| Gruppe - | | Aufnahme (mg/14 d) | | faecale Exkretion (mg/ 14 d) | | scheinbare Absorption (mg/14 d) | | scheinbare Absorption (%) |
|---|---|---|---|---|---|---|---|---|
| Ia, 10 ppm zn (Sulfat) | M<br>SD | 1,76<br>±0,305 | *b* | 0,411<br>±0,122 | *a* | 1,35<br>±0,280 | bc | 76,4<br>±7,33 |
| Ib, 10 ppm Zn (Lipoat) | M<br>SD | 1,68<br>±0,106 | *b* | 0,445<br>±0,080 | *a* | 1,24<br>±0,092 | b | 73,6<br>±4,11 |
| IIa, 10 ppm Zn (Sulfat) + PA | M<br>SD | 0,836<br>±0,146 | *a* | 0,484<br>±0,162 | *a* | 0,351<br>±0,038 | a | 43,0<br>±8,66 |
| IIb, 10 ppm Zn (Lipoat) + PA | M<br>SD | 0,941<br>±0,099 | *a* | 0,494<br>±0,059 | *a* | 0,446<br>±0,078 | a | 47,3<br>±5,23 |
| IIIa, 20 ppm Zn (Sulfat) + PA | M<br>SD | 2,57<br>±0,334 | *c* | 1,43<br>±0,170 | *b* | 1,13<br>±0,219 | b | 43,9<br>±4,63 |
| IIIb, 20 ppm Zn (Lipoat) + PA | M<br>SD | 3,30<br>±0,308 | *d* | 1,71<br>±0,231 | *b* | 1,60<br>±0,199 | c | 48,4<br>±4,55 |

Bei Verfütterung PA-freier Diäten mit suboptimaler Zn-Konzentration (Gruppen Ia und Ib) wurde eine hohe Zn-Einlagerung in das Femurgewebe der Ratten ersichtlich (Tabelle 16). Nach Zulage von 0,4 % PA (Gruppen IIa und IIb) hingegen wurde eine drastische und signifikante Reduktion der Zn-Gehalte in den Knochen festgetellt. Nach einer Verdopplung der diätetischen Zn-Stufe (Gruppen IIIa und IIIb) wurde ein signifikanter Anstieg der Zn-Konzentration in den Femora auf ein mittleres Niveau nachgewiesen. Zn-Lipoat zeigte im Vergleich zu Zn-Sulfat bei Phytatzulage einen positiven Effekt auf die Höhe der Zn-Einlagerung in das Femurgewebe.

Vergleichbare Ergebnisse konnten auch für die Zn-Konzentration im Plasma aufgezeigt werden. So lag die Plasma-Zn-Konzentration bei Verfütterung PA-freier Diäten mit niedriger Zn-Stufe auf einem hohen Niveau, wohingegen bei Zulage von 0,4 % PA die Plasma-Gehalte signifikant reduziert wurden. Ein erneuter, teilweise signifikanter Anstieg dieser Werte wurde bei einer Verdopplung der diätetischen Zn-Konzentration auf 20 mg je kg Diät ersichtlich.

Auch hinsichtlich der Aktivität der Alkalischen Phosphatase im Plasma konnten vergleichbare Entwicklungen aufgezeigt werden. So lag die Aktivität des Zn-abhängigen Enzyms bei Verfütterung PA-freier Diäten trotz niedrigem Zn-Gehalt der Diäten auf einem hohen Niveau, wohingegen bei Zulagen von 0,4 % PA ein signifikanter Abfall der Aktivität bei den Gruppen IIa und IIb ersichtlich wurde. Nach Erhöhung der diätetischen Zn-Konzentration auf 20 mg je kg Diät wurde eine Steigerung der Enzymaktivität auf ein gegenüber den Gruppen Ia und Ib vergleichbares Niveau verzeichnet.

Tabelle 16 zeigt die Zn-Konzentrationen in Femur und Plasma sowie Aktivität der alkalischen Phosphatase im Plasma wachsender Ratten bei Zulage von Zn-Sulfat, Zn-Lipoat und Phytinsäure (PA) (n = 6 x 6). Unterschiedliche Hochbuchstaben innerhalb einer Spalte kennzeichnen signifikante Differenzen von mind. $p < 0,05$ (Tukey-HSD)

Tabelle 16

| Gruppe | | Femur (µg/g FM) | | Plasma (µg/ml) | | Alkalische Phosphatase (mU/ml Plasma) | |
|---|---|---|---|---|---|---|---|
| Ia, 10 ppm Zn (Sulfat) | M | 81,4 | c | 1,11 | bc | 383 | b |
| | SD | ±4,60 | | ±0,24 | | ±52,0 | |
| Ib, 10 ppm Zn (Lipoat) | M | 79,2 | c | 1,24 | c | 365 | b |
| | SD | ±8,00 | | ±0,24 | | ±54,6 | |
| IIa, 10 ppm Zn (Sulfat) + PA | M | 36,3 | a | 0,57 | a | 216 | a |
| | SD | ±4,74 | | ±0,18 | | ±53,4 | |
| IIb, 10 ppm Zn (Lipoat) + PA | M | 41,2 | a | 0,50 | a | 225 | a |
| | SD | ±4,72 | | ±0,15 | | ±38,1 | |
| IIIa, 20 ppm Zn (Sulfat) + PA | M | 55,4 | b | 0,80 | ac | 347 | b |
| | SD | ±9,53 | | ±0,25 | | ±42,0 | |
| IIIb, 20 ppm Zn (Lipoat) + PA | M | 64,1 | b | 0,96 | bc | 384 b | |
| | SD | ±10,1 | | ±0,18 | | ±62,8 | |

Bei den gemessenen Metallothionein-Konzentration im Lebergewebe ergaben sich in allen Gruppen (I, II and III) nominelle Steigerungen der hepatischen MT-Konzentration, wenn die Zn-Quelle Zn-Lipoat und nicht $ZnSO_4$ war.

Tabelle 17 zeigt die Metallothionein-Konzentration im Lebergewebe wachsen-der Ratten bei Zulage von Zn-Sulfat, Zn-Lipoat und Phytinsäure (PA) (n = 6 x 6).

Tabelle 17

| Gruppe | | Metallothionein (ng MT/mg Protein) |
|---|---|---|
| Ia, 10 ppm Zn (Sulfat) | M | 8,08 |
| | SD | ±2,71 |
| Ib, 10 ppm Zn (Lipoat) | M | 8,32 |
| | SD | ±4,16 |
| IIa, 10 ppm Zn (Sulfat) + PA | M | 6,36 |
| | SD | ±4,66 |
| IIb, 10 ppm Zn (Lipoat) + PA | M | 7,94 |
| | SD | ±2,29 |
| IIIa, 20 ppm Zn (Sulfat) + PA | M | 7,58 |
| | SD | ±3,15 |
| IIIb, 20 ppm Zn (Lipoat) + PA | M | 8,71 |
| | SD | ±6,46 |

**Patentansprüche**

1. Verwendung von α-Liponsäure oder α-Dihydroliponsäure zur Erhöhung der Bioverfügbarkeit von Mineralsalzen.

2. Verwendung nach Anspruch 1, **dadurch gekennzeichnet, daß** man mindestens ein Mineralsalz in Kombination mit α-Liponsäure oder α-Dihydroliponsäure verwendet.

3. Verwendung nach Anspruch 2, **dadurch gekennzeichnet, daß** man Mineralsalze der Formel I verwendet,

$$(M)_n(B)_m \qquad I$$

wobei

M ein 1- bis 3-wertiges, physiologisch verträgliches Metallkation,

B ein 1- bis 3-wertiges, physiologisch verträgliches Anion,

n 1, 2 oder 3 und

m 1, 2 oder 3 bedeuten,

wobei die Indizes n und m dem Wertigkeits- und Ladungsausgleich des Mineralsalzes der Formel I entsprechen.

4. Verwendung nach Anspruch 1 oder 2, **dadurch gekennzeichnet, daß** man als Kombination Metall-α-Lipoate, Metall-α-Dihydrolipoate oder Metall-α-Liponsäure-Komplexe verwendet.

5. Verwendung nach Anspruch 4, **dadurch gekennzeichnet, daß** man als Kombination Metall-α-Lipoate, Metall-α-Dihydrolipoate oder Metall-α-Liponsäure-Komplexe der Formel II verwendet,

$$(M)_w(Lp)_x(A)_y(H_2O)_z \qquad II$$

wobei

M ein 1 bis 3-wertiges, physiologisch verträgliches Metallkation oder ein Gemisch 1- bis 3-wertiger, physiologisch verträglicher Metallkationen,
Zeichn.

Lp racemische α-Liponsäure oder α-Dihydroliponsäure, (R)- oder (S)-α-Liponsäure oder (R)- oder (S)-α-Dihydroliponsäure, racemisches α-Lipoat oder Dihydro-α-lipoat oder (R)- oder (S)-α-Lipoat oder (R)- oder (S)-Dihydro-α - lipoat,

A ein physiologisch verträgliches, ein oder zweiwertiges Anion,

w 1 oder 2

x 1, 2, 3 oder 4,

y 0, 1, 2 oder 3 und

z 0, 1, 2, 3, 4, 5 oder 6, bedeuten,

wobei die Indizes w, x und y dem Wertigkeits- und Ladungsausgleich der Verbindung der Formel II entsprechen.

6. Verwendung nach einem der Ansprüche 1 bis 5, **dadurch gekennzeichnet, daß** man als α-Liponsäure (R)-α-Liponsäure oder als α-Lipoat (R)-α-Lipoat verwendet.

7. Zubereitung, enthaltend mindestens ein Mineralsalz und (R)-α-Liponsäure oder (S)-α-Liponsäure.

**8.** Metall-α-Lipoate, Metall-α-Dihydrolipoate oder Metall-α-Liponsäure-Komplexe der Formel II'

$$(M)_w(Lp)_x(A)_y(H_2O)_z \qquad II'$$

wobei

M ein 1 bis 3-wertiges, physiologisch verträgliches Metall kation oder ein Gemisch 1- bis 3-wertiger, physiologisch verträglicher Metallkationen,
Lp racemische α-Liponsäure oder α-Dihydroliponsäure, (R)-oder (S)-α-Liponsäure oder (R)- oder (S)-α-Dihydroliponsäure, racemisches α-Lipoat oder Dihydro-α-lipoat oder (R)- oder (S)-α-Lipoat oder (R)- oder (S)-Dihydro-α-lipoat,
A ein physiologisch verträgliches, ein oder zweiwertiges Anion,
w 1 oder 2
x 1, 2, 3 oder 4,
y 0, 1, 2 oder 3 und
z 0, 1, 2, 3, 4, 5 oder 6, bedeuten,

wobei die Indizes w, x und y dem Wertigkeits- und Ladungsausgleich entsprechen und
folgende Verbindungen ausgenommen sind:
$Mn(Lip^-)ClO_4$, $Cu(Lip^-)ClO_4$, $Zn(Lip^-)ClO_4$, $Cd(Lip^-)ClO_4$, $Pb(Lip^-)ClO_4$, $Hg(Lip_{rac})$ $(OH)_2$, $Hg(DHL_{rac}{}^{2-})$, Ni $(DHL_{rac}{}^{2-})$, $Fe_2(DHL_{rac}{}^{2-})_3$, $Zn(Lip_{rac}{}^-)_2(H_2O)_2$, $Cd(Lip_{rac}{}^-)_2(H_2O)_2$
wobei

Lip - einwertig negatives, racemisches oder (R)- oder (S)-α-Lipoat,

$Lip_{rac}$ - einwertig negatives, racemisches α-Lipoat,

$Lip_{rac}$ - racemische α-Liponsäure und

$DHL_{rac}{}^2$ - zweiwertig negatives, racemisches α-Dihydrolipoat bedeuten.

**9.** Zubereitung, enthaltend Metall-α-Lipoate, Metall-α-Dihydrolipoate oder Metall-α-Liponsäure-Komplexe gemäß Anspruch 8.

**10.** Zubereitung, enthaltend Metall-α-Lipoate, Metall-α-Dihydrolipoate oder Metall-α-Liponsäure-Komplexe gemäß Anspruch 5 und α-Liponsäure oder α-Dihydroliponsäure.

**11.** Verwendung der Metall-α-Lipoate, Metall-α-Dihydrolipoate oder Metall-α-Liponsäure-Komplexe gemäß Anspruch 5 als Futter-oder Nahrungsergänzungsmittel.

**12.** Verwendung der Metall-α-Lipoate, Metall-α-Dihydrolipoate oder Metall-α-Liponsäure-Komplexe gemäß Anspruch 5 in kosmetischen Formulierungen.

**13.** Metall-α-Lipoate, Metall-α-Dihydrolipoate oder Metall-α-Liponsäure-Komplexe gemäß Anspruch 5 zur Verwendung als Arzneimittel.

**14.** Verwendung der Metall-α-Lipoate, Metall-α-Dihydrolipoate oder Metall-α-Liponsäure-Komplexe gemäß Anspruch 5 zur Herstellung eines Arzneimittels zur Behandlung von Krankheiten bei denen Liponsäure einen therapeutischen oder prophylaktischen Effekt hat und ein Mineralsalzmangel vorliegt.

**15.** Verwendung der Metall-α-Lipoate, Metall-α-Dihydrolipoate oder Metall-α-Liponsäure-Komplexe gemäß Anspruch 5 nach Anspruch 14 zur Behandlung von Diabetes, Tumorerkrankungen, HIV-Infektionen, AIDS, Niereninsuffizienz, Mangelernährung, Proteinenergiemalnutrition sowie Mineralstoffmmangelzuständen.

Abb. 1:

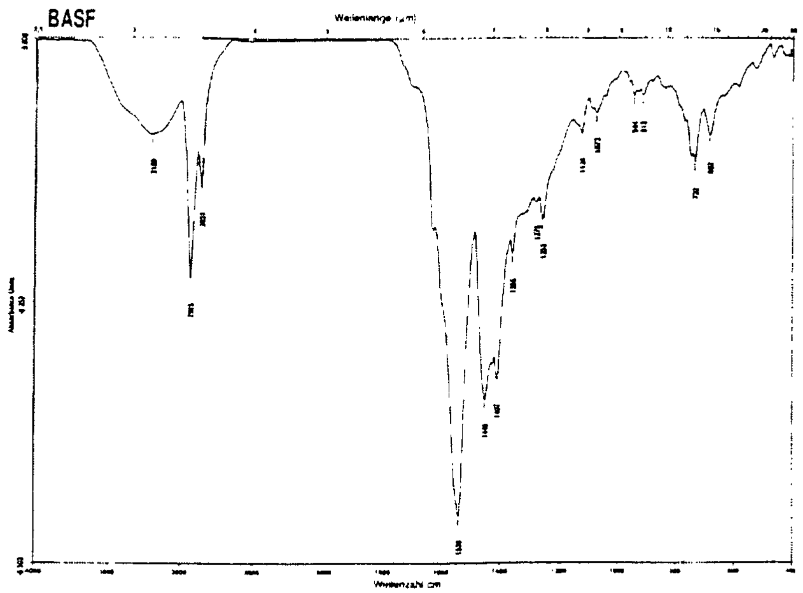

Abb. 2:

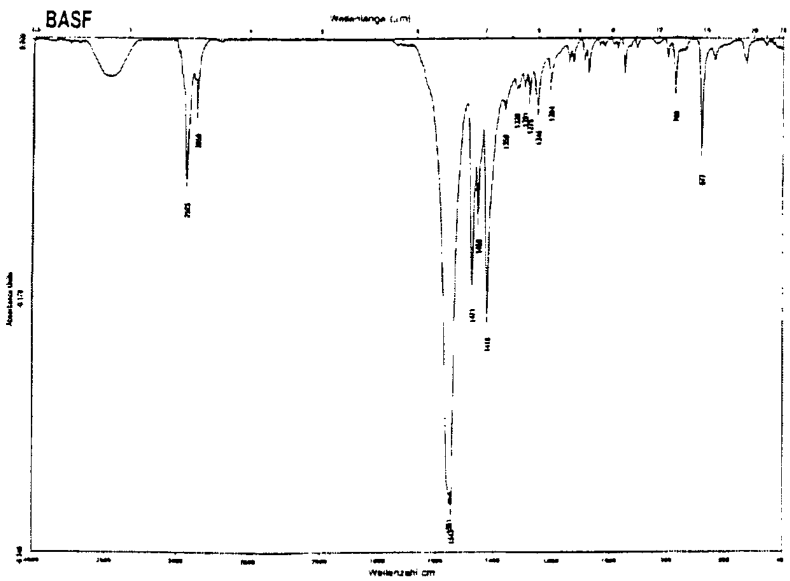

Abb. 3:

3a

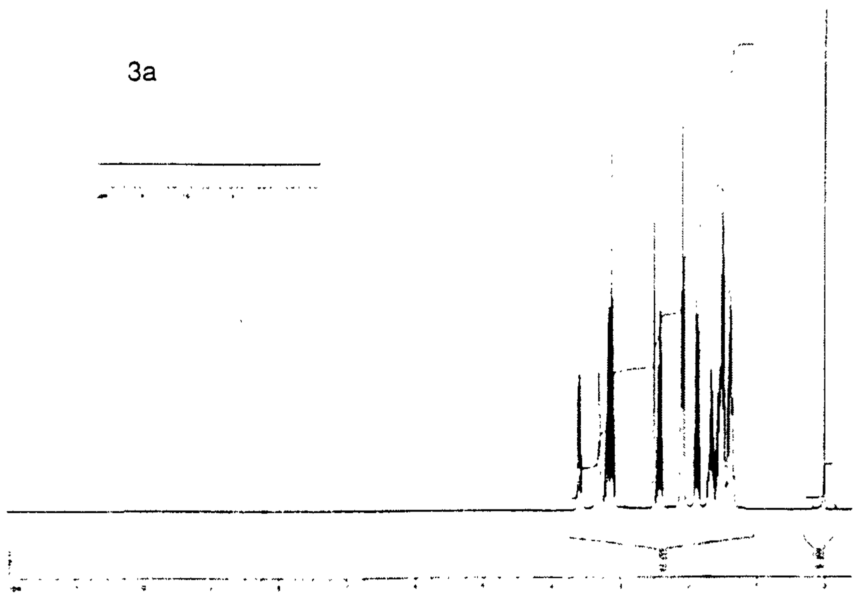

3b